# EUROPÄISCHE PATENTSCHRIFT

(11) **EP 0 775 682 B1**
(45) Veröffentlichungstag und Bekanntmachung des Hinweises auf die Patenterteilung: **03.03.1999**
(21) Anmeldenummer: 96118575.8
(22) Anmeldetag: 20.11.1996
(51) Int. Cl.: C07C 13/40, C07C 5/29

(54) **Verfahren zur Herstellung von Camphen durch Umlagerung von alpha-Pinen**
Process for the preparation of camphene by rearrangement of alpha-pinene
Procédé pour la préparation du camphène par transposition de l'alpha-pinène

(30) Priorität: 27.11.1995 DE 19544086
(43) Veröffentlichungstag der Anmeldung: 28.05.1997
(73) Patentinhaber: Clariant GmbH, 65929 Frankfurt am Main (DE)
(72) Erfinder: Gscheidmeier, Manfred, Dr., 86456 Gablingen (DE); Häberlein, Harald, Dr., 86356 Neusäss (DE)

(56) Entgegenhaltungen:
- EP-A- 0 539 990
- US-A- 2 551 795

## Beschreibung

Die vorliegende Erfindung betrifft ein verbessertes Verfahren zur Herstellung von Camphen mit einem gegenüber bisher bekannten Methoden stark reduzierten Gehalt an isomerem Tricyclen, bei dem gleichzeitig die Bildung von hochsiedenden Terpenpolymeren weitestgehend vermieden wird.

Die Bildung von Camphen durch Isomerisation von α-Pinen mittels heterogener Katalyse ist seit langem bekannt (L.G. Gurvich, J. Russ. Phys,. Chem. Soc., 47, 827/1915). Hierzu wurden Katalysatoren vielfältiger Art vorgeschlagen, wobei sich saures Titandioxidhydrat besonders bewährt hat (Zusammenfassung: Ullmanns Encyclopädie der technischen Chemie, 3. Auflage, Band 17, 1966). Bei der Reaktion entstehen ab ca. 125°C neben dem Hauptprodukt Camphen mehrere Nebenprodukte wie Tricyclen, Dipenten, isomere p-Menthadiene und Polymere.

In geringem Umfang treten auch Cyclofenchen, α-Fenchen, Bornylen, Δ-3-Caren, p-Cymol, p-Menthen-3 und Diterpene auf.

Dieses Rohcamphen wird durch fraktionierende Vakuumdestillation zu technisch reinem Camphen aufgearbeitet, welches üblicherweise auch das Tricyclen enthält, für das es - in isoliertem Zustand - keine technische Verwertungsmöglichkeit gibt. Tricyclen reagiert chemisch weitgehend wie Camphen, kann aber auch zu isomeren Produkten führen, die stören würden.

Camphen spielt eine bedeutende Rolle als Zwischenprodukt, z. B. zur Herstellung von Terpenestern (preiswerte und häufig naturidentische Riechstoffe, wie Isobornylacetat, oder Monomere für Lackharze), Terpensauerstoff-Verbindungen (Isoborneol, Kampfer), Terpenäthern oder Alkylierungsprodukten (z.B. Phenolen). Dabei ist es häufig von Bedeutung, daß der Tricyclengehalt im Camphen möglichst unter 10 % liegt.

Die Isomerisationsreaktion ist stark exotherm, es muß also Wärme abgeführt werden. Dies kann z.B. durch direkte Kühlung oder Umpumpen über ein Kühlaggregat erfolgen, um die Temperatur unterhalb des Siedebereiches zu halten. Dies hat den Vorteil des geringen Katalysatorbedarfs (0,05 - 0,1 Gew.-%) und etwas höherer Camphenausbeute bei gleichzeitiger Vermeidung der Bildung hochsiedender Nebenprodukte. Allerdings benötigt die Reaktion dann den 10 - 15fachen Zeitaufwand im Vergleich zu einer Reaktionsführung am Produktsiedepunkt. In diesem Fall wird die Reaktionswärme durch Verdampfen von α-Pinen bzw. von Reaktionsprodukten mit anschließender Kondensation abgeführt, d.h. man läßt das Reaktionsgemisch am Rückfluß kochen (C.A. Vol. 89/1978, 197 725 g; EP-A-0 539 990). Unter diesen Bedingungen (156 - 162°C, 0,2 - 0,4 Gew.-% Katalysator) läuft die Isomerisation rasch in 0,75 - 2 Stunden, aber besonders in der Endphase weniger selektiv ab, hauptsächlich durch verstärkte Bildung von Terpenpolymeren aus p-Menthadienen.

Es wurde nun gefunden, daß bei der Isomerisation von α-Pinen in Gegenwart von schwach saurem Titandioxidhydrat als Katalysator beim Kochen am Rückfluß und mit Stickstoff-Überlagerung zunächst keine Polymeren gebildet werden; dies tritt erst dann ein, wenn der Restgehalt an α-Pinen auf 5 %, verstärkt auf 3 % abgesunken ist. Dies erfolgt normalerweise, wenn die Siedetemperatur deutlich über 161,5°C ansteigt. Dann nimmt der Anteil an hochsiedenden polymeren Verbindungen auf Kosten der p-Menthadiene zeitabhängig zu bis über 5 %.

Diese Polymerisationsreaktion läßt sich nun vermeiden, indem man bei Erreichen eines bestimmten α-Pinen-Gehaltes die Reaktionstemperatur absenkt und bei dieser Temperatur die Reaktion zu Ende führt. Man erreicht hiermit die rasche Reaktion durch Kochen am Rückfluß, vermeidet aber durch Absenken der Temperatur in der Endphase die Bildung von hochsiedenden polymeren Produkten weitestgehend.

Gegenstand der Erfindung ist somit ein Verfahren zum Herstellen von Camphen durch Umlagerung von α-Pinen in Gegenwart eines Titanoxidhydrat-Katalysators in der Wärme, wobei man α-Pinen zunächst solange unter Rückfluß erhitzt, bis der Gehalt an α-Pinen im Reaktionsgemisch noch mindestens 3, vorzugsweise 5 Gew.-% beträgt, und anschließend in einer Nachreaktion die Umlagerung bei einer Temperatur unter 160°C, vorzugsweise bei 150 - 155°C zu Ende führt.

Das Verfahren kann im einzelnen in der Weise durchgeführt werden, daß das α-Pinen und der Katalysator in einen Rührkessel eingefüllt und unter Abdeckung mit Stickstoff vorzugsweise mit einer Aufheizgeschwindigkeit von 0,5 bis 10, vorzugsweise 2 bis 4°C/min auf Rückflußtemperatur erhitzt wird. Dabei wird die Reaktionswärme durch Siedekühlung abgeführt. Die Rückflußtemperatur, d.h. der Siedepunkt von α-Pinen bzw. des im Laufe der Umlagerung entstehenden Reaktionsgemisches hängt von der Reinheit des α-Pinen ab. Im allgemeinen liegt die Rückflußtemperatur bei ca. 155 bis 165°C. Die Reaktionszeit beträgt 0,3 bis 3,5, vorzugsweise 0,7 bis 1,5 h je nach Katalysatorkonzentration und Aufheizgeschwindigkeit.

Der Katalysator ist übliches Titanoxidhydrat, vorzugsweise ein Titanoxidhydrat, welches folgendermaßen gewonnen wird:
Eine schwefelsaure TiO₂-Paste wird mit NaOH-Lösung versetzt, dann gewaschen und mit Essigsäure gerührt, abgenutscht und anschließend bei 50 bis 100, vorzugsweise bei 60 bis 80°C im Vakuum getrocknet.

Die einzusetzende Menge an Katalysator beträgt 0,1 bis 2,0, vorzugsweise 0,3 bis 0,5 Gew.-%, bezogen auf reines α-Pinen.

Wenn die Rückflußtemperatur erreicht ist,wird das Reaktionsgemisch solange bei dieser Temperatur gehalten, bis der Gehalt an α-Pinen im Reaktionsgemisch auf ca. 3 Gew.-%, vorzugsweise auf ca. 5 Gew.-% abgesunken ist. Die Abnahme des Gehaltes an α-Pinen im Verlauf der Umlagerungsreaktion läßt sich leicht durch geeignete analytische Methoden, beispielsweise durch Gaschromatographie, verfolgen. Wenn diese Restmengen an α-Pinen im Reaktionsgemisch erreicht sind, wird die Innentemperatur des Reaktionsgemisches abgesenkt und die Umwandlung des noch vorhandenen α-Pinens in einer Nachreaktion bei tieferen Temperaturen zu Ende geführt. Die Temperatur in dieser Nachreaktion hängt ab von der Siedetemperatur des Reaktionsgemisches. Sinnvoll ist es, die Temperatur bei der Nachreaktion um ca. 10°C unter die Siedetemperatur des Reaktionsgemisches zu senken. Dies bedeutet im allgemeinen eine Temperatur von ca. 150 - 155°C. In jedem Fall soll die Temperatur unter 160°C liegen. Die Dauer der Nachreaktion hängt ab von dem jeweils gewünschten Restgehalt an α-Pinen. Wenn die gewünschte Restmenge, beispielsweise 0,1 Gew.-% an α-Pinen, erreicht ist, wird das Reaktionsgemisch abgekühlt, vom Katalysator abfiltriert und das Camphen wird durch Destillation von den Nebenprodukten abgetrennt.

Gemäß einer Variante des erfindungsgemäßen Verfahrens nimmt man einen weniger aktiven Titanoxidhydrat-Katalysator oder eine geringere Menge eines aktiveren Katalysators dieser Art. Die Hauptreaktion erfordert dann zwar etwas mehr Zeit, doch fällt hier die Reaktionstemperatur gegen Ende des α-Pinen-Isomerisierung von selbst unter die Siedetemperatur, so daß man für die Nachreaktion auf eine aktive Kühlung verzichten kann.

Bei der Isomerisierung von α-Pinen entsteht neben dem Camphen immer Tricyclen, wobei der Anteil an Tricyclen von der Reaktionstemperatur abhängt. Bei einer Reaktionstemperatur von 155°C erhält man beispielsweise 13,6 Gew.-% Tricyclen im Reaktionsgemisch bzw. 17,1 Gew.-% in der von p-Menthadien befreiten Fraktion. Es wurde nun gefunden, daß sich dieser Gleichgewichtswert auch dann einstellt, wenn man ein Produktgemisch mit hohem Tricyclengehalt den Isomerisationsbedingungen wie bei α-Pinen unterwirft. Der Gleichgewichtswert stellt sich hier wesentlich rascher ein als bei Isomerisation eines Gemisches mit entsprechend geringerem Tricyclengehalt. Da bei der destillativen Aufarbeitung des α-Pinen-Isomerisationsproduktes im Vakuum wegen des etwas höheren Dampfdruckes von Tricyclen dieses in einem ersten Destillationsabschnitt stark angereichert ist (bis über 50 %), besteht nun die Möglichkeit, diesen, mit Tricyclen angereicherten ersten Destillationsabschnitt abzutrennen und erneut einem Isomerisierungs-Ansatz mit α-Pinen zuzuführen. Es stellt sich dann erneut das Gleichgewichtsverhältnis am jeweiligen Siedepunkt ein, was bedeutet, daß entweder ein über dem am Siedepunkt herrschenden Gleichgewichtswert liegender Tricyclenanteil in Camphen umgewandelt wird oder bei darunter liegenden Tricyclengehalten nur so viel Tricyclen neu gebildet wird, bis der Gleichgewichtswert erreicht ist. Bei mit Tricyclenangereicherten Fraktionen ergibt sich damit die Möglichkeit, daß Tricyclen in Camphen umgewandelt wird. Selbstverständlich ist es auch möglich, den tricyclenreichen Destillations-Abschnitt allein den Isomerisationsbedingungen wie bei α-Pinen zu unterwerfen. Vorteilhaft ist, daß hierbei keine p-Menthadiene und somit auch keine hochsiedenden Nebenprodukte auftreten. Als Folge der Abtrennung eines tricyclenreichen ersten Abschnittes aus der Rohcamphen-Destillation erhält man eine Camphen-Hauptfraktion, die nun im Durchschnitt wesentlich weniger Tricyclen enthält, je nach Tricyclengehalt und Menge des ersten Abschnittes bis weit unter 10 %.

Das erfindungsgemäße Verfahren ermöglicht es somit, den Anteil an polymeren Nebenprodukten bei der Umwandlung von α-Pinen in Camphen zu senken und damit den Anteil an Camphen zu erhöhen. Außerdem ist es möglich, durch die Abtrennung einer ersten tricyclenreichen Fraktion bei der destillativen Aufarbeitung des Reaktionsgemisches und Rückführung dieser angereicherten Fraktion in die Isomerisierungsreaktion den Anteil an Tricyclen im Camphen zu verringern.

### Beispiel 1 (Vergleichsversuch)

### Pinen-Isomerisation ohne Zugabe von Tricyclen-Vorlauf; Temperatur steigt über 162°C:

In einem 5 m³-Rührwerksbehälter mit Doppelmantel wurden unter ständiger Stickstoff-Überlagerung 3038 kg techn. α-Pinen (ca. 96 % α-Pinen, ca. 1,5 β-Pinen, ca. 1 % Camphen) und 12 kg schwach saures Titandioxidhydrat unter Rühren auf 155°C erhitzt. Infolge der exothermen Isomerisationsreaktion stieg die Temperatur des Reaktionsgemisches weiter bis zum Siedepunkt des α-Pinens bzw. der Isomerisationsprodukte. Die Produktdämpfe wurden kondensiert, das Kondensat ohne weitere Abkühlung in den Reaktor zurückgeführt. Als nach ca. 60 Min. der α-Pinengehalt auf 0,1 % abgenommen hatte, wurde die Reaktion durch Kühlung beendet, die Innentemperatur lag zu diesem Zeitpunkt bei 163,1°C mit leicht steigender Tendenz. Nach Filtration des Reaktionsgemisches ergab die Analyse 7,2 % hochsiedende polymere Produkte, die ausschließlich über p-Menthadiene entstanden sind, und Tricyclen (T), Camphen (C) und p-Menthadiene (p-M) (hauptsächlich α-Terpinen, γ-Terpinen, Dipenten, Terpinolen) in Gewichtsverhältnis T:C:p-M = 1:4,3:0,9.

### Beispiel 2

### Pinen-Isomerisation ohne Zusatz von Tricyclen-Vorlauf

Analog zu Beispiel 1 wurden 2994 kg α-Pinen und 7,5 kg schwach saures Titandioxidhydrat zur Reaktion am Siedepunkt gebracht. Die Reaktionstemperatur stieg dabei bis knapp 162°C und begann dann leicht zu fallen. Nach ca. 80 Min. wurde abgekühlt. Das Reaktionsgemisch enthielt nur 0,1 Gew.-% hochsiedende polymere Produkte; das Gewichtsverhältnis von Tricyclen, Camphen und p-Menthadien betrug 1:4,8:1,8.

### Beispiel 3

### Pinen-Isomerisation mit Zusatz von Tricyclen-Vorlauf

Analog zu Beispiel 1 wurden 3000 kg α-Pinen und 350 kg Tricyclen-Vorlauf (mit 49 % Tricyclen) mit 7,5 kg schwach saurem Titandioxidhydrat zur Reaktion gebracht. Die Reaktionstemperatur stieg bis 161,2°C und begann dann leicht zu fallen. Nach ca. 120 Min. wurde die Reaktion durch Abkühlen abgebrochen. Das Reaktionsgemisch enthielt nur 0,2 Gew.-% hochsiedende polymere Anteile; das Gewichtsverhältnis von Tricyclen, Camphen und p-Menthadien betrug 1:4,5:1,3.

### Beispiel 4

### Pinen-Isomerisation mit Zusatz von Tricyclen-Vorlauf.

Analog zu Beispiel 1 wurden 3022 kg α-Pinen und 309 kg Tricyclen-Vorlauf (mit 51 % Tricyclen) mit 10 kg schwach saurem Titandioxidhydrat zur Reaktion gebracht. Nachdem die Reaktionstemperatur bis auf 161,7°C gestiegen und der α-Pinen-Gehalt unter ca. 3 % gefallen war, wurde das Reaktionsgemisch auf 150°C abgekühlt und bei dieser Temperatur noch ca. 1 Std. gerührt. Nach Reaktionsende, d.h. bei einem Restgehalt an α-Pinen von unter 0,1 Gew.-%, wurde das Reaktionsgemisch abgekühlt. Das Reaktionsgemisch war völlig frei von hochsiedenden polymeren Nebenprodukten; das Gewichtsverhältnis Tricyclen, Camphen und p-Menthadien betrug 1:4,6:1,5.

## Patentansprüche

1. Verfahren zur Herstellung von Camphen durch Umlagerung von α-Pinen in Gegenwart eines Titanoxidhydrat-Katalysators in der Wärme, dadurch gekennzeichnet, daß man α-Pinen solange unter Rückfluß erhitzt, bis der Gehalt an α-Pinen im Reaktionsgemisch noch mindestens 3 Gew.-% beträgt, und anschließend in einer Nachreaktion die Umlagerung bei einer Temperatur unter 160°C zu Ende führt.

2. Verfahren nach Anspruch 1, dadurch gekennzeichnet, daß man das α-Pinen solange unter Rückfluß erhitzt, bis der Gehalt an α-Pinen im Reaktionsgemisch noch mindestens 5 Gew.-% beträgt.

3. Verfahren nach Anspruch 1, dadurch gekennzeichnet, daß man die Umlagerung bei einer Temperatur von 150 bis 155°C zu Ende führt.

4. Verfahren nach Anspruch 1, dadurch gekennzeichnet, daß man in der Nachreaktion die Temperatur von unter 160°C durch Kühlung des Reaktionsgemisches einstellt.

5. Verfahren nach Anspruch 1, dadurch gekennzeichnet, daß man eine an Tricyclen angereicherte Fraktion, die man bei der destillativen Aufarbeitung des Reaktionsgemisches erhält, in das α-Pinen zurückführt.

## Claims

1. A process for the preparation of camphene by the rearrangement of α-pinene in the presence of a titanium oxide hydrate catalyst under the action of heat, wherein α-pinene is refluxed until the content of α-pinene in the reaction mixture is still at least 3% by weight, and the rearrangement is then brought to completion in a secondary reaction at a temperature below 160°C.

2. The process as claimed in claim 1, wherein the α-pinene is refluxed until the content of α-pinene in the reaction mixture is still at least 5% by weight.

3. The process as claimed in claim 1, wherein the rearrangement is brought to completion at a temperature of 150 to 155°C.

4. The process as claimed in claim 1, wherein the temperature in the secondary reaction is brought below 160°C by cooling of the reaction mixture.

5. The process as claimed in claim 1, wherein a tricyclene-enriched fraction, obtained from the distillative working-up of the reaction mixture, is recycled into the α-pinene.

## Revendications

1. Procédé de préparation à chaud de camphène par transposition d'α-pinène en présence d'un catalyseur en hydrate d'oxyde de titane, caractérisé en ce que, l'on chauffe l'α-pinène à reflux jusqu'à ce que la teneur en α-pinène dans le mélange réactionnel soit encore d'au moins 3% en poids, et qu'ensuite on termine la transposition par une réaction ultérieure à une température inférieure à 160°C.

2. Procédé selon la revendication 1, caractérisé en ce que, l'on chauffe l'α-pinène à reflux jusqu'à ce que la teneur en α-pinène dans le mélange réactionnel soit encore d'au moins 5% en poids.

3. Procédé selon la revendication 1, caractérisé en ce que l'on termine la transposition à une température de 150 à 155°C.

4. Procédé selon la revendication 1, caractérisé en ce que l'on ajuste dans la réaction ultérieure la température à une valeur inférieure à 160°C par refroidissement du mélange réactionnel.

5. Procédé selon la revendication 1, caractérisé en ce que l'on réintroduit dans l'α-pinène une fraction enrichie en tricyclène, que l'on obtient au cours du traitement par distillation du mélange réactionnel.
